# FASCICULE DE BREVET EUROPEEN

(11) **EP 1 434 622 B1**
(45) Date de publication et mention de la délivrance du brevet: **04.04.2007**
(21) Numéro de dépôt: 02790508.2
(22) Date de dépôt: 11.10.2002
(51) Int. Cl.: A61N 1/30

(54) **DISPOSITIF DE DELIVRANCE DE MEDICAMENTS PAR IONTOPHORESE TRANSPALPEBRALE**
VORRICHTUNG ZUR IONTOPHORETISCHEN VERABREICHUNG VON MEDIKAMENTEN DURCH DAS AUGENLID
DEVICE FOR DELIVERING MEDICINES BY TRANSPALPEBRAL ELECTROPHORESIS

(30) Priorité: 12.10.2001 FR 0113176
(43) Date de publication de la demande: 07.07.2004
(73) Titulaire: Eyegate Pharma SA, 75012 Paris (FR)
(72) Inventeur: BEHAR, Francine, F-75016 Paris (FR); ROY, Pierre, F-75019 Paris (FR)
(74) Mandataire: Callon de Lamarck, Jean-Robert
(86) Numéro de dépôt international: PCT/FR2002/003472
(87) Numéro de publication internationale: WO 2003/030989

(56) Documents cités:
- EP-A- 1 127 586
- WO-A-99/40967
- US-A- 4 564 016
- US-A- 6 001 088
- US-A- 6 154 671

## Description

L'invention concerne des dispositifs de délivrance de principes actifs notamment par iontophorèse transpalpébrale.

La iontophorèse utilise le courant électrique pour permettre la diffusion d'une molécule ionisée à travers une membrane biologique à des fins thérapeutiques. Sous l'effet du courant électrique, la perméabilité de la membrane biologique est augmentée, ce qui permet le passage de molécules plus importantes au sein de la cellule, et le champ électrique pousse les molécules à travers cette membrane. Cette technique présente l'avantage par rapport à une application topique classique d'augmenter la profondeur :de pénétration du principe actif proportionnellement à l'intensité de courant utilisée et au temps d'application.

Les techniques actuelles de délivrance de principes actifs au niveau de l'oeil peuvent être classées en cinq catégories décrites comme suit :
- la voie systémique (voie orale ou voie intraveineuse), parfois par bolus (forte dose, durée courte) ne donne pas des concentrations élevées dans l'oeil (moins de 1%) car les vaisseaux sanguins de la rétine et d'autres parties du système nerveux central sont relativement imperméables à de nombreux principes actifs. De plus, les drogues utilisées par voie systémique peuvent avoir des effets secondaires importants sur d'autres organes du corps que celui explicitement visé,
- les injections directes autour de l'oeil dans les espaces périoculaires ou dans le vitré sont très traumatisantes. De plus, après injection dans le vitré, la drogue est rapidement diluée et disparaît en quelques heures. Ce mode d'administration présente certains risques comme les risques d'infection, de saignement, de cataracte ou de détachement de la rétine. Ces problèmes peuvent être partiellement résolus par des implants intraoculaires de libération programmée de principes actifs implantés directement dans le vitré, implants solides ou semi-solides, suturés ou non qui nécessitent une intervention chirurgicale pour leur implantation et une deuxième intervention pour le retrait de ceux faits de matériaux non biodégradables,
- les applications topiques par gouttes ne s'appliquent qu'à des produits actifs que l'on peut formuler en collyre (solution ou suspension) et ne peuvent pas traiter le segment postérieur du globe oculaire car la pénétration du principe actif est très limitée et ne permet pas d'atteindre des concentrations thérapeutiques au delà du segment antérieur du globe oculaire. D'autre part, comme les larmes lavent la drogue rapidement, les applications doivent être répétées fréquemment. Il est possible de palier ces problèmes en utilisant un insert conjonctival permettant une libération programmée de principes actifs, tout en augmentant le temps de contact du principe actif avec la surface oculaire. Cette solution présente l'inconvénient d'avoir une tolérance médiocre et un bénéfice faible car il s'avère impossible d'obtenir des concentrations élevées de principe actif dans le segment postérieur de l'oeil (rétine, nerf optique),
   la thérapie photodynamique est une technique qui consiste à injecter de façon systémique un principe actif et à l'activer de façon locale en utilisant un laser présentant une certaine longueur d'onde. L'inconvénient majeur de cette thérapie est que le patient doit rester dans l'obscurité plusieurs jours, le principe actif doit être modifié par l'ajout d'agents, l'un photosensible bloquant son activité jusqu'à ce que le laser procède à son activation, l'autre qui permet sa fixation sur des composés biologiques spécifiques du corps humain. Cette modification supporte donc que le principe actif soit entièrement testé pharmaceutiquement avant sa mise sur le marché, même s'il s'agit d'un médicament connu. Enfin le médecin doit disposer d'un matériel relativement onéreux en l'acquisition d'un laser spécifique,
- enfin, il existe des dispositifs de iontophorèse oculaire comme celui indiqué dans le brevet US-A-6 154 671 qui décrit un dispositif de iontophorèse dont le réservoir comportant le principe actif vient en contact direct avec le globe oculaire. Un tel dispositif permet d'obtenir des concentrations et des temps de résidence intraoculaire égaux ou supérieurs aux techniques précédemment citées tout en étant non invasif. Cependant, la technique présentée dans ce document reste d'une utilisation délicate pour des traitements de long terme et ne peut pas être utilisée quand la surface du globe oculaire est affectée.

Dans le domaine de l'anesthésie régionale, en dehors de l'anesthésie générale nécessaire pour des durées longues ou pour des patients non collaboratifs, il existe quatre techniques qui concernent le globe oculaire :
- l'anesthésie topique consiste à instiller de façon topique l'anesthésique dans les cul-de-sacs conjonctivaux. Cette technique donne une anesthésie courte mais suffisante pour de nombreuses opérations, mais de moins bonne qualité, car il y a plus de mobilité oculaire, et elle entraîne un accroissement des douleurs postopératoires. Il est fréquent lors de l'utilisation de cette technique de faire usage de sédatifs administrés de façon intraveineuse et pouvant entraîner des complications comme un arrêt respiratoire. Dans ce cas, la présence d'un anesthésiste est vivement recommandée,
- l'anesthésie rétrobulbaire consiste à injecter l' anesthésique à l'aide d'une aiguille à l'arrière du globe oculaire, à l'intérieur de l'espace formé par les muscles oculomoteurs. Cette technique d'anesthésie présente des risques de perforation du globe oculaire lui-même, des risques d'hémorragie rétrobulbaire, des risques de blessures du nerf optique, des risques d'arrêt cardiaque ou respiratoire, des risques d'injection intravasculaire accidentelle de l'anesthésique, ainsi que des risques d'occlusion vasculaire rétinienne. Cependant la qualité et la durée de l'anesthésie est bonne,
- l'anesthésie péribulbaire consiste à injecter l'anesthésique à l'aide d'une aiguille du globe oculaire et à l'extérieur de l'espace formé par les muscles oculomoteurs. Cette technique conduit aux mêmes complications que la précédente mais de manière moins fréquente car la pénétration de l'aiguille est moins profonde,
- enfin, l'anesthésie rétrobulbaire par cathéter est une technique consistant à mettre en place un cathéter de type péridurale (entre 0,4mm et 1mm de diamètre) à l'aide d'une aiguille, au sein de l'espace rétrobulbaire ou péribulbaire de sorte à pouvoir injecter l'anesthésique pour des opérations de longue durée ou bien de l'administrer en continu même en post-opératoire. Les risques entraînés par cette technique d'anesthésie sont identiques aux deux précédentes.

Un but de la présente invention est de fournir un dispositif de délivrance de principes actifs oculaires d'un emploi très simple et capable de cibler les zones anatomiques de l'oeil à traiter tout en étant non invasif.

Pour cela, on prévoit, selon l'invention, un dispositif d'application oculaire d'un principe actif comprenant une électrode principale comportant une couche isolante, une couche adhésive apte à lier la couche isolante à une couche conductrice, l'électrode principale présentant en outre une zone apte à venir en contact avec une paupière d'un oeil.
Ainsi, l'électrode principale est placée directement sur la paupière de l'oeil à traiter. Elle permet ainsi de traiter la zone de la sclère qui est la plus perméable et qui présente le moins de risque pour la vision (car il n'y a pas de rétine fonctionnelle à l'intérieur de l'oeil autour de la cornée). D'autre part, l'électrode fonctionnelle ne vient pas en contact direct avec le globe oculaire. Le dispositif s'appuie sur le fait que l'épaisseur de la peau au niveau de la paupière est la moins importante de l'organisme. Ainsi, le patient peut utiliser le dispositif seul sans nécessiter la présence d'un médecin, ce qui est avantageux pour des iontophorèses de très longue durée devant être pratiquées (jusqu'à 18 heures). Cela permet de traiter des pathologies des annexes oculaires et des glandes des paupières. Les anomalies des glandes palpébrales sont responsables d'anomalies ou de diminution de la qualité du film lacrymal, responsables des pathologies de la surface oculaire.

Avantageusement, le dispositif d'application oculaire d'un principe actif présente au moins l' une des caractéristiques suivantes :
- l'électrode principale est de forme générale ovale,
- la forme ovale de l'électrode présente un grand diamètre extérieur au plus égal à environ 40mm et un petit diamètre extérieur au plus égal à environ 35mm,
- la zone présente une zone centrale non fonctionnelle entourée par une zone périphérique fonctionnelle,
- la zone centrale non fonctionnelle est de forme circulaire,
- la forme circulaire de la zone centrale non fonctionnelle présente un diamètre au plus égal à environ 13mm,
- la zone centrale non fonctionnelle est un orifice traversant l'électrode,
- l'électrode principale est souple,
- l'électrode principale présente en plus une couche d'adhésif cutané,
- l'électrode principale présente en plus une couche de mousse liée à la couche conductrice par une couche d'adhésif conducteur,
- la couche de mousse est une couche absorbante apte à faire office de réservoir pour le principe actif,
- la couche isolante est une coque rigide, et
- le principe actif est appliqué par iontophorèse.

On prévoit aussi selon l'invention une électrode comportant une couche isolante et une couche adhésive apte à lier la couche isolante à une couche conductrice, caractérisée en ce qu'elle présente une zone centrale non fonctionnelle entourée par une zone périphérique fonctionnelle apte à venir en contact avec une paupière de l'oeil.

On prévoit aussi, selon l'invention une méthode d'application oculaire d'un principe actif comprenant des étapes de pose d'un réservoir de médicaments comportant une électrode principale sur les paupières, de pose d'une électrode de retour sur les tissus adjacents au globe oculaire à traiter, de pénétration à travers la paupière du principe actif sous l'effet d'un courant d'énergie circulant entre les électrodes.

Avantageusement, la méthode présente une des caractéristiques suivantes :
- préalablement à la pose de l'électrode active sur les paupières le principe actif est disposé sous les paupières,
- l'électrode principale présente au moins l'une des caractéristiques précitées,
- le principe actif est sous une forme topique (liquide, suspension, gel),
- le principe actif est sous une forme d'insert.

D'autres caractéristiques et avantages de l'invention apparaîtront à la description d'un mode de réalisation et de variantes. Aux dessins annexés :
- la figure 1 est une représentation schématique d'un dispositif d'application d'un principe actif oculaire selon l'invention,
- la figure 2 est une représentation en vue de dessus d'une électrode principale selon l'invention,
- la figure 3 est une représentation schématique en coupe de l'électrode principale de la figure 2,
- la figure 4 est une représentation schématique en coupe de l'électrode principale de la figure 2 selon une variante de réalisation,
- les figures 5a et 5b sont une représentation d'une première variante de réalisation d'un dispositif d'application d'un principe actif oculaire selon l'invention,
- les figures 6a, 6b et 6c sont une représentation d'une deuxième variante de réalisation d'un dispositif d'application d'un principe actif oculaire selon l'invention, et
- la figure 7 est une représentation d'une troisième variante de réalisation d'un dispositif d'application d'un principe actif oculaire selon l'invention.

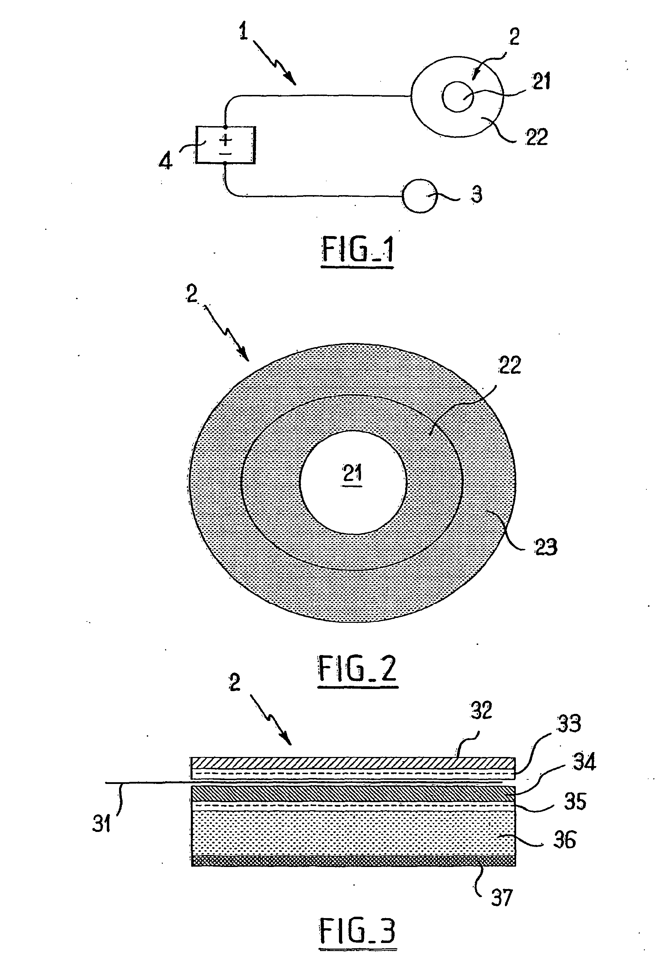

En référence à la figure 1, nous allons décrire le dispositif d'application oculaire d'un principe actif par iontophorèse selon l'invention. Le dispositif 1 comprend un générateur de courant 4 relié d'une part à une électrode de retour 3 et d'autre part à une électrode principale 2.

Le générateur de courant 4 délivre un courant continu compris entre 0,5mA et 5mA de préférence, voire jusqu'à 10mA, et cela pendant un temps compris entre environ 0,5 et environ 30 minutes de préférence, voire jusqu'à 18 heures environ. En fonction de la résistance électrique des tissus formant le circuit, résistance susceptible d'évoluer pendant la iontophorèse, la tension délivrée par le générateur s'adapte selon la loi d'Ohm, U = R.I, où U est la tension en Volts et R la résistance totale du circuit en Ohms et I l'intensité choisie en Ampères. Cependant la tension délivrée par ce générateur de courant ne pourra jamais excéder 80V. Il peut être envisagé d'utiliser un générateur de courant alternatif de façon à éviter une augmentation de pH sous l'effet de phénomène d'oxydo-réduction au niveau de l'électrode, notamment en cas de traitement prolongé. La plage de fréquences de ces courants est choisie afin de permette une augmentation maximale de la perméabilité des tissus du principe actif. Dans ce cas particulier, l'électrode de retour 3 doit être de type électrocardiogramme et composée d'un adhésif et d'un film Ag/AgCl d'impédance faible. Enfin le générateur peut utiliser un profil de courant présentant des pics de tension très élevés, compris entre 50 et 2500V environ, sur de très courtes durées de l'ordre de 0.01 à 0.1 seconde, et ce, sous faible intensité (comme ceux décrit pour l'électroporation).

Il est possible d'utiliser d'autres modes permettant d'améliorer la perméabilité des membranes biologiques : la magnétophorèse qui utilise les champs magnétiques, l'énergie électromagnétique de la radiofréquence et des micro-ondes, l'énergie ultrasonore. De préférence, un dispositif selon l'invention utilise la iontophorèse ou l'électroporation.

En référence à la figure 2, la forme générale de l'électrode principale 2 est ovale. De préférence, son grand diamètre extérieur correspond sensiblement au grand diamètre de l'orbite oculaire, soit environ 40mm. De même, son petit diamètre extérieur correspond au petit diamètre de l'orbite, soit environ 35mm. Ces dimensions correspondent à la taille adulte courante de l'orbite. D'autres tailles et formes peuvent être envisagées en fonction de l'âge et de la morphologie du patient à traiter.

L'électrode 2 présente une :zone non fonctionnelle 21 centrale. De préférence, cette zone centrale non fonctionnelle sera de forme circulaire dont le diamètre correspond sensiblement au diamètre de la cornée, soit environ 13mm. De manière préférentielle, l'électrode comporte un orifice central traversant faisant office de zone non fonctionnelle 21.

La zone périphérique fonctionnelle entourant la zone non fonctionnelle centrale présente deux sous-zones 22 et 23. La sous-zone fonctionnelle 22 est apte à venir en regard de la surface de la sclère située autour de la cornée. De même, la sous-zone fonctionnelle 23 est apte à couvrir la surface de la paupière où se situent les attaches des muscles oculomoteurs.

Cette forme particulière de l'électrode principale 2 permet, lors de l'utilisation, de couvrir le maximum de surface de la sclère autour de la cornée et le maximum de surface de la paupière où se situent les attaches des muscles oculomoteurs. Il est à noter que la surface de la sclère située autour de la cornée est la plus perméable et présente le moins de risque pour la vision car il n'y a pas de rétine à l'intérieur de l'oeil autour de ladite cornée.

De manière générale l'électrode principale 2 est souple. Ainsi, l'électrode principale 2 se conforme à la paupière lors de sa mise en place de manière à épouser le plus intimement possible les tissus palpébraux et à permettre, ainsi, un bon contact électrique avec la paupière.

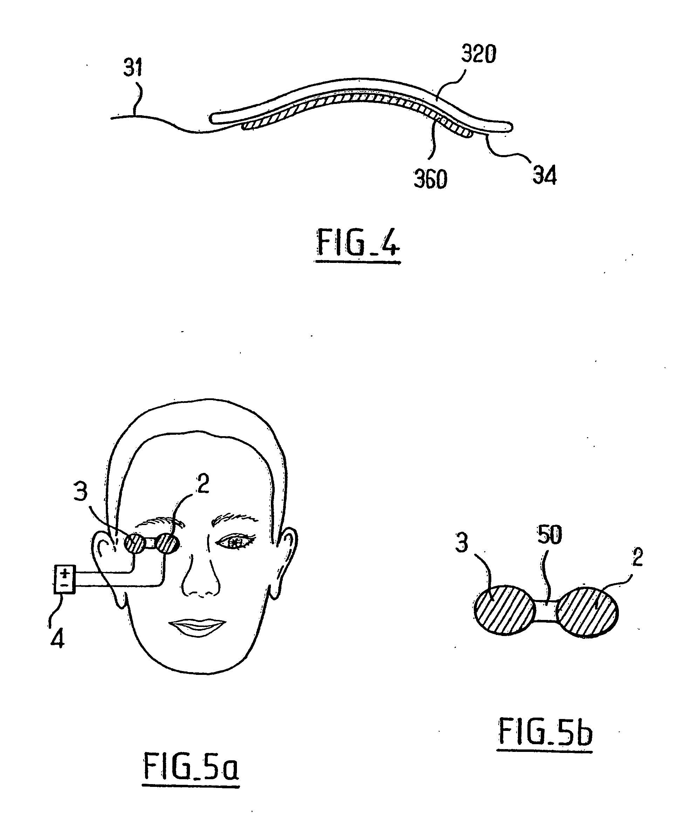

Dans un autre mode de réalisation représenté à la figure 4, l'électrode 2 peut être rigide. Dans ce cas, elle se présente sous la forme d'une coque 320 dont la face interne est revêtue d'un matériau souple 360 apte à absorber les différences anatomiques, de manière à épouser le plus intimement possible les tissus palpébraux et de permettre ainsi un bon contact électrique.

En référence à la figure 3 nous allons décrire la structure de l'électrode. Nous y avons représenté, en coupe, les six couches de matériau pouvant constituer l'électrode, certaines de ces couches étant optionnelles comme nous allons le voir. Chacune de ces couches de matériau a une fonction précise.

La première couche 32 est une couche isolante. C'est la partie de l'électrode principale 2 qui est apte à être en contact avec l'opérateur. Elle permet d'isoler le reste de l'électrode de ce dernier. Cette couche peut être souple et plate ou bien rigide et en forme de coque.

La couche 33 est une couche adhésive qui assure la fonction de .liaison entre la couche isolante 32, une couche conductrice 34 décrite ci-après. D'autre part, cette couche adhésive 33 permet de maintenir sur la couche conductrice 34 un cordon électrique 31 reliant l'électrode principale 2 au générateur 4.

La couche 34 est une couche conductrice. Cette couche est composée d'un film d'argent et d'un film de carbone et a pour rôle de répartir le courant électrique sur toute la surface de la zone fonctionnelle de l'électrode principale 2. Le film d'argent de cette couche conductrice est située en regard de la couche adhésive 33. Il permet une bonne répartition du courant sur la surface du film de carbone et assure un contact électrique optimum avec le cordon électrique 31. De son côté, le film de carbone est disposé en regard d'une couche de mousse absorbante 36 décrite ci-après. Cette couche de carbone résiste à l'oxydation en milieu aqueux sous un courant électrique continu. La matériau le plus adapté est du film Argent/Carbone d'épaisseur 0,2mm (Rexam conductive film référence 2252 de Rexam Image Products).

La couche 36 est une couche optionnelle. C'est une couche de mousse absorbante apte à être imprégnée par le principe actif ou par une solution comprenant le principe actif avant utilisation. De ce fait, cette couche de mousse absorbante devra être très absorbante et comprendre des pores de petites dimensions de l'ordre de 100 à 500 micromètres. Le matériau le plus adapté est, par exemple, de la mousse polyuréthanne hydrophile à cellules ouvertes de faible densité de l'ordre de 0,05 à 0,1g/cm³ (Hydrocrest™ de Crest Foam Ind, Capu-cell^{®} de TMP Technologies inc., Amrel^{®} de Rynel, Medicell™ Foam d'Hydromer). Cette couche est facultative selon que le principe actif est placé dans la mousse avant utilisation ou placé directement sous la paupière avant la mise en place de l'électrode sur la paupière.

Cette couche de mousse absorbante 36 est liée sur le film de carbone de la couche conductrice 34 par une couche d'adhésif conducteur 35. Cet adhésif conducteur ne doit pas être soluble dans l'eau.

Enfin, la couche 37 est une couche d'adhésif cutané. Cette couche est facultative selon que l'électrode principale est adhésive ou non. Le type d'adhésif choisi devra être conducteur du courant électrique d'une part et devra permettre le passage du principe actif tout en adhérant le moins possible à la peau afin de pouvoir être enlevé facilement après utilisation. Cette couche d'adhésif cutané peut être située sur la couche de mousse absorbante si celle-ci est présente ou bien directement sur le film de carbone de la couche conductrice 34.

Une des variantes de réalisation consiste à remplacer les couches adhésives 33, conductrice 34 et d'adhésif conducteur 35 par une seule couche d'adhésif conducteur (ARcare^{®} 8881 de Adhesive Research Inc.) qui remplit avantageusement toutes les fonctions précitées.

Le premier mode de réalisation de l'électrode principale 2 est une électrode souple comportant une couche d'adhésif cutané 37, une couche conductrice 34, une couche d'adhésif 33 et une couche isolante 32.

Le deuxième mode de réalisation de l'-électrode principale 2 est une électrode souple comportant une couche d'adhésif cutané 37, une couche de mousse absorbante 36, une couche d'adhésif conducteur 35, une couche conductrice 34., une couche d'adhésif 33 et une couche isolante 32.

Le troisième mode de réalisation de l'électrode principale 2 est une électrode rigide en forme de coque comportant une couche d'adhésif cutané 37, une couche de mousse 36 faisant office de matériau souple destiné à absorber les différences anatomiques et à épouser plus intimement possible les tissus palpébraux lors de l'utilisation, une couche d'adhésif conducteur 35, une couche conductrice 34, une couche d'adhésif 33 et une couche isolante 32 rigide formant la coque.

Enfin, le quatrième mode de réalisation de l'électrode principale 2 est identique au troisième mode de réalisation ci-dessus décrit, la couche de mousse étant remplacée par la couche de mousse absorbante 36.

Nous allons maintenant décrire l'utilisation de l'électrode et de son dispositif selon l'invention.
Dans le cadre des premier et troisième modes de réalisation de l'électrode principale 2, l'opérateur, qui peut être un médecin ou le patient lui-même, dispose le principe actif ou une solution comprenant le principe actif sous la paupière de l'oeil à traiter puis pose l'électrode principale 2 sur la paupière et l'électrode de retour 3 sur les tissus adjacents du globe oculaire à traiter. Les électrodes sont ensuite reliées au générateur et l'ensemble du circuit est mis sous tension selon une intensité définie et un temps d'application défini. Ensuite, les électrodes sont retirées.

Dans le cas des deuxième et quatrième modes de réalisation de l'électrode principale 2, l'opérateur imbibe la couche de mousse absorbante avec le principe actif ou une solution comprenant le principe actif. Puis positionne l'électrode principale sur la paupière du globe oculaire à traiter et pose l'électrode de retour sur les tissus environnant le globe oculaire à traiter. Ensuite l'opérateur effectue les mêmes opérations que précédemment décrites.

Les différents médicaments ou principes actifs aptes à être administrés par un dispositif selon l'invention sont ceux nécessitant une application régulière ou sur une longue période. C'est le cas, par exemple, des corticostéroïdes ou anti-inflammatoires non stéroïdiens (dexamethazone, methylprednisolone hemisuccinate, etc...) dont l'administration doit être prolongée sur de très longues périodes dans les cas d'inflammation chronique.

On trouve aussi les anti-allergiques, les neuroprotecteurs, les neuromodulateurs, les anti-glaucomateux, les antibiotiques, les anti-angiogéniques, les facteurs neurotropiques, les anesthésiques. De nombreuses autres molécules sont en cours de développement pour freiner voire arrêter la néovascularisation observée dans les pathologies dégénératives de la rétine. Les molécules peuvent être transférées par voie transclérale par iontophorèse ou par injection vitréenne puis iontophorèse. Le dispositif de iontophorèse selon l'invention permet de simplifier l'administration de ces médicaments comme nous avons pu le voir ci-dessus.

Une autre indication du dispositif d'application d'un principe actif selon l'invention concerne l'anesthésie locale de la paupière ou bien des muscles oculomoteurs situés dans l'orbite, qui sont au nombre de six et qui permettent les mouvements de rotation de l'oeil. Il s'agit du muscle droit interne, du muscle droit externe, du muscle droit supérieur, du muscle droit inférieur, du grand oblique et du petit oblique. A ceux-là, s'ajoutent le muscle releveur de la paupière et le muscle orbiculaire (peut avoir un intérêt pour la chirurgie esthétique, le traitement des exophtalmi ou des ptosis). Cette indication permet d'immobiliser le paupière et/ou une akinésie de l'oeil dans le cadre des opérations de chirurgie.

D'autres utilisations du dispositif d'application oculaire d'un principe actif selon l'invention concernent les principes actifs ne nécessitant pas une application récurrente mais qui ne peuvent pas être administrés par un dispositif ayant un contact cornéen ou scléral direct pour une raison physiologique comme, par exemple, un traumatisme de la surface du globe oculaire ou suite à des séquelles d'ordre chirurgical. Parmi les principes actifs cernés, on peut citer les antibiotiques, les antiviraux, les anti-inflammatoires ou les antifongiques par exemple.

Bien entendu, on pourra apporter de nombreuses modifications à l'invention sans pour autant sortir du cadre de celle-ci.

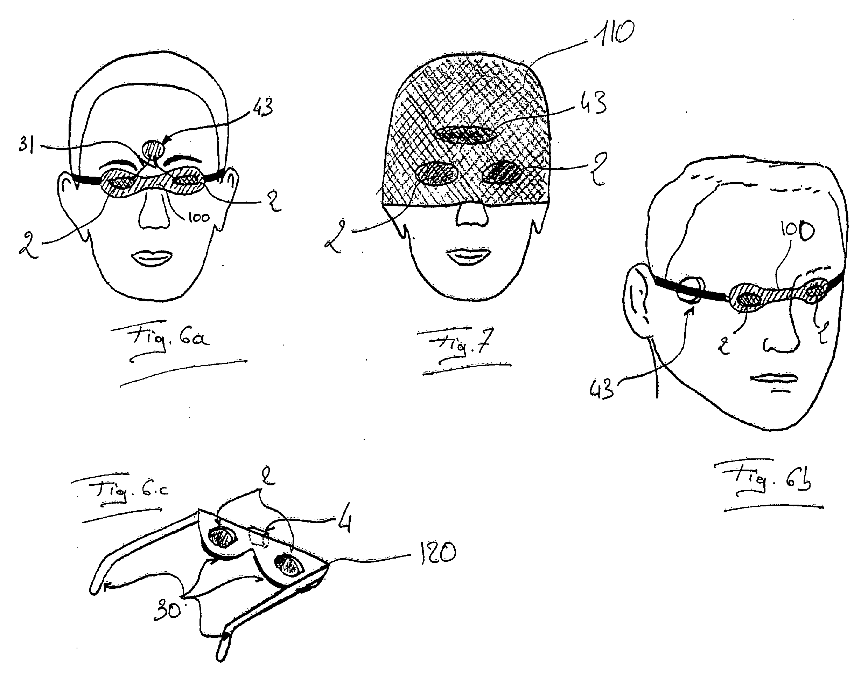

Par exemple :
- L'électrode de retour 3 disposée sur les tissus adjacents au globe oculaire peut être reliée à l'électrode principale 2 par l'intermédiaire d'un film non conducteur 50 et disposée sur la tempe du patient à traiter comme illustré aux figures 5a et 5b.
- Les électrodes principales 2 peuvent être au nombre de deux (une pour chaque oeil) et disposées sur un masque 100 (tel qu'un masque de nuit utilisé dans les transports aériens par exemple), l'électrode de retour 3 associée au générateur (pièce 43) peut être placée sur le front du patient de manière préférentielle, et reliée aux électrodes principales par l'intermédiaires des cordons électriques 31, comme cela est illustré en figure 6a. Il est à noter que le masque 100 est maintenu sur la tête du patient par des moyens élastiques tel un bandeau.
- Dans une variante illustrée en figure 6b de l'agencement précédemment décrit, une électrode de retour et le générateur (pièce 43) peuvent être placés de façon avantageuse à l'intérieur du bandeau élastique de maintien du masque 100 comportant les deux électrodes principales 2 et ce de manière à ce que l'électrode de retour soit sur l'une des tempes (ou bien les deux dans une variante de disposition où il y aurait deux électrodes de retour). Ici, le moyen élastique de maintien sert également de cordon électrique qui est réalisé sous la forme de pistes conductrices par exemple.
- En figure 6c est illustrée une paire de lunette 120 comportant deux électrodes principales 2 situées en lieu et place des verres de la paire de lunette 120. Un générateur 4 est installé sur la monture de la paire de lunette 120 et relié, d'une part, aux deux électrodes 2 et, d'autre part, aux électrodes de retour 30 qui sont, de préférence, situées sous les électrodes principales 2 de manière à être en contact (tout en étant isolées de ces électrodes principales) avec les peaux du haut des joues du patient ou situées sur les extrémités des branches de la paire de lunette 120 de manière à être en contact avec la peau située derrière les oreilles du patient.
- En figure 7 est illustrée une autre variante de réalisation dans laquelle les deux électrodes principales 2 sont montées sur un bonnet 110 ainsi que la pièce 43 comportant le générateur et l'électrode de retour.

Dans les quatre dernières variantes illustrées aux figures 6a, 6b, 6c et 7, l'électrode de retour peut être placée sur les tissus du visage environnant le globe oculaire comme les tempes, le front, les joues etc..., de manière générale. D'autre part, le générateur peut y être associé ou non. Il est possible, dans des variantes, de déporter le générateur relié aux électrodes principales 2 et de retour 3 par l'intermédiaire de fils électriques. Dans le cas contraire, les fils de raccordement sont intégrés au dispositif sous la forme de pistes conductrices par exemple.

Ces quatre modes de réalisation sont bien adaptés pour traiter des pathologies dégénératives comme la rétinopathie diabétique, la dégénérescence maculaire liée à l'âge, la rétinite pigmentaire. En effet, il est connu, pour ces pathologies, que la probabilité que les deux yeux soient atteints est élevée (de l'ordre de 50% des cas). Dans ce cas, il faut soigner les deux yeux en même temps ou sur un même intervalle de temps de façon alternative.

## Revendications

1. Dispositif d'application oculaire d'un principe actif (1) comprenant une électrode principale (2) comportant une couche isolante (32), une couche d'adhésif (33) apte à lier la couche isolante à une couche conductrice (34), l'électrode principale présentant une zone (21,22,23) apte à venir en contact avec une paupière d'un oeil.

2. Dispositif selon la revendication 1, **caractérisé en ce que** l'électrode principale présente une forme générale ovale.

3. Dispositif selon la revendication 2, **caractérisé en ce que** la forme ovale présente un grand diamètre extérieur qui est au plus égal à environ 40mm et un petit diamètre extérieur qui est au plus égal à environ 35mm.

4. Dispositif selon l'une des revendications 1 à 3, **caractérisé en ce que** la zone présente une zone centrale non fonctionnelle (21) entourée par une zone périphérique fonctionnelle (22,23)

5. Dispositif selon la revendication 4, **caractérisé en ce que** la zone centrale non fonctionnelle est de forme générale circulaire.

6. Dispositif selon la revendication 5, **caractérisé en ce que** la zone centrale non fonctionnelle présente un diamètre au plus égal à environ 13mm.

7. Dispositif selon l'une des revendications 4 à 6, **caractérisé en ce que** la zone centrale non fonctionnelle est un orifice traversant l'électrode principale.

8. Dispositif selon l'une des revendications précédentes, **caractérisé en ce que** l'électrode principale présente en outre une couche d'adhésif cutané (37).

9. Dispositif selon l'une des revendications précédentes, **caractérisé en ce que** l'électrode principale présente en outre une couche de mousse (36) liée à la couche conductrice par une couche d'adhésif conducteur (35).

10. Dispositif selon la revendication 9, **caractérisé en ce que** la couche de mousse (36) est une couche de mousse absorbante apte à faire office de réservoir pour le principe actif.

11. Dispositif selon l'une des revendications précédentes, **caractérisé en ce que** l'électrode principale est souple.

12. Dispositif selon l'une des revendications 1 à 10, **caractérisé en ce que** la couche isolante est une coque rigide.

13. Dispositif selon l'une des revendications 1 à 12, **caractérisé en ce que** le principe actif est applicable par iontophorèse.

14. Electrode pour l'application oculaire d'un principe actif comportant une couche isolante et une couche adhésive apte à lier la couche isolante à une couche conductrice (34), cette électrode présentant une zone apte à venir en contact avec une paupière de l'oeil.

## Claims

1. A device for ocular application of an active substance (1) comprising a main electrode (2) comprising an insulating layer (32) and an adhesive layer (33) able to bond the insulating layer to a conductive layer (34), the main electrode having an area (21, 22, 23) able to come into contact with an eyelid.

2. A device according to Claim 1,
**characterised in that** the main electrode is of oval overall shape.

3. A device according to Claim 2,
**characterised in that** the oval shape has a large external diameter which is equal at most to approximately 40mm and a small external diameter which is equal at most to approximately 35mm.

4. A device according to one of Claims 1 to 3, **characterised in that** the area has a non-functional central area (21) surrounded by a functional peripheral area (22, 23).

5. A device according to Claim 4,
**characterised in that** the non-functional central area is of circular overall shape.

6. A device according to Claim 5,
**characterised in that** the non-functional central area has a diameter equal at most to approximately 13mm.

7. A device according to one of Claims 4 to 6, **characterised in that** the non-functional central area is a hole passing through the main electrode.

8. A device according to one of the preceding claims, **characterised in that** the main electrode has in addition a cutaneous adhesive layer (37).

9. A device according to one of the preceding claims, **characterised in that** the main electrode has in addition a foam layer (36) bonded to the conductive layer by a conductive adhesive layer (35).

10. A device according to Claim 9,
**characterised in that** the foam layer (36) is an absorbent foam layer able to act as a reservoir for the active substance.

11. A device according to one of the preceding claims, **characterised in that** the main electrode is flexible.

12. A device according to one of Claims 1 to 10, **characterised in that** the insulating layer is a rigid casing.

13. A device according to one of Claims 1 to 12, **characterised in that** the active substance can be applied by iontophoresis.

14. An electrode for the ocular application of an active substance comprising an insulating layer and an adhesive layer able to bond the insulating layer to a conductive layer (34), such electrode having an area able to come into contact with an eyelid.

## Patentansprüche

1. Vorrichtung zur Anwendung eines Wirkstoffs (1) am Auge, die eine Hauptelektrode (2) umfaßt, die folgendes umfaßt: eine isolierende Schicht (32) und eine Haftschicht (33), die dafür eingerichtet ist, die isolierende Schicht mit einer leitenden Schicht (34) zu verbinden, wobei die Hauptelektrode eine Zone (21, 22, 23) hat, die dafür eingerichtet ist, mit einem Augenlid in Kontakt zu kommen.

2. Vorrichtung nach Anspruch 1, **dadurch gekennzeichnet, daß** die Hauptelektrode eine allgemein ovale Form hat.

3. Vorrichtung nach Anspruch 2, **dadurch gekennzeichnet, daß** die ovale Form einen großen Außendurchmesser hat, der höchstens etwa 40 mm beträgt, und einen kleinen Außendurchmesser, der höchstens etwa 35 mm beträgt.

4. Vorrichtung nach einem der Ansprüche 1 bis 3, **dadurch gekennzeichnet, daß** die Zone eine nicht funktionelle zentrale Zone (21) hat, die von einer funktionellen Randzone (22, 23) umgeben ist.

5. Vorrichtung nach Anspruch 4, **dadurch gekennzeichnet, daß** die nicht funktionelle zentrale Zone allgemein kreisförmig ist.

6. Vorrichtung nach Anspruch 5, **dadurch gekennzeichnet, daß** die nicht funktionelle zentrale Zone einen Durchmesser von höchstens etwa 13 mm hat.

7. Vorrichtung nach einem der Ansprüche 4 bis 6, **dadurch gekennzeichnet, daß** die nicht funktionelle zentrale Zone eine Öffnung ist, die durch die Hauptelektrode verläuft.

8. Vorrichtung nach einem der vorhergehenden Ansprüche, **dadurch gekennzeichnet, daß** die Hauptelektrode außerdem eine Hauthaftschicht (37) hat.

9. Vorrichtung nach einem der vorhergehenden Ansprüche, **dadurch gekennzeichnet, daß** die Hauptelektrode außerdem eine Schaumstoffschicht (36) hat, die mit der leitenden Schicht durch eine leitende Haftschicht (35) verbunden ist.

10. Vorrichtung nach Anspruch 9, **dadurch gekennzeichnet, daß** die Schaumstoffschicht (36) eine Schicht aus absorbierendem Schaumstoff ist, die dafür geeignet ist, als Speicher für den Wirkstoff zu dienen.

11. Vorrichtung nach einem der vorhergehenden Ansprüche, **dadurch gekennzeichnet, daß** die Hauptelektrode biegsam ist.

12. Vorrichtung nach einem der Ansprüche 1 bis 10, **dadurch gekennzeichnet, daß** die isolierende Schicht eine steife Schale ist.

13. Vorrichtung nach einem der Ansprüche 1 bis 12, **dadurch gekennzeichnet, daß** der Wirkstoff durch lontophorese anwendbar ist.

14. Elektrode zur Anwendung eines Wirkstoffs am Auge, die folgendes umfaßt: eine isolierende Schicht und eine Haftschicht, die dafür eingerichtet ist, die isolierende Schicht mit einer leitenden Schicht (34) zu verbinden, wobei diese Elektrode eine Zone hat, die dafür geeignet ist, mit einem Augenlid in Kontakt zu kommen.
